# EUROPEAN PATENT SPECIFICATION

(11) **EP 4 100 752 B1**
(45) Date of publication and mention of the grant of the patent: **07.10.2026**
(21) Application number: 21750094.1
(22) Date of filing: 03.02.2021
(51) Int. Cl.: G01R 31/28, A61M 16/16

(54) **RESPIRATORY OR SURGICAL HUMIDIFIER AND METHOD OF USE**
ATMUNGS- ODER OPERATIONSBEFEUCHTER UND VERWENDUNGSVERFAHREN
HUMIDIFICATEUR D'AIR INHALÉ OU CHIRURGICAL ET PROCÉDÉ D'UTILISATION

(30) Priority: 04.02.2020 US 202062970047 P
(43) Date of publication of application: 14.12.2022
(73) Proprietor: Fisher & Paykel Healthcare Limited, East Tamaki Auckland 2013 (NZ)
(72) Inventor: YU, Yintao, Auckland, 2013 (NZ); ALNASHI, Sinaa, Auckland, 2013 (NZ); LIANG, Wenjie Robin, Auckland, 2013 (NZ); TSANG, Jonathan Luke Hoi-Man, Auckland, 2013 (NZ)
(74) Representative: Treeby, Philip David William
(86) International application number: PCT/NZ2021/050013
(87) International publication number: WO 2021/158127

(56) References cited:
- EP-A2- 2 775 277
- US-A1- 2009 223 514
- US-A1- 2010 116 272
- US-A1- 2014 216 459
- US-B2- 8 416 546
- US-B2- 9 952 286

## Description

### FIELD OF THE DISCLOSURE

The present disclosure relates to respiratory and/or surgical humidifiers, and respiratory or breathing assistance systems for gases to be supplied to a patient or user via a gas supply tube incorporating a tube heating element.

### BACKGROUND

Respiratory and/or surgical humidifiers are used in various environments, such as hospital, medical facility, residential care, palliative care and home environments. For a range of respiratory applications, it is beneficial to humidify gases being supplied to a patient or user. These applications include where the gases are for breathing by the patient or user and where the gas is being supplied during surgery to the patient or user.

In the case of noninvasive ventilation therapy (e.g. with a face or nasal mask), inhalation of dry breathing gasses can dry out the tissues of the patient or user's airway (e.g., the nasal mucosa). Humidifying the breathing gasses prior to inspiration can mitigate drying of the patient or user's airway tissue. Furthermore, this humidification can increase the patient or user's comfort, and improve the patient or user's tolerance to the noninvasive ventilation (NIV).

In the case of high flow therapy, humidified gases are delivered to the patient or user at high flows through an unsealed interface. The patient or user may be spontaneously breathing or may be apneic, such as under anesthesia. As with noninvasive ventilation, humidifying the gases inspired during high flow therapy can mitigate drying of the airway tissue, increase comfort, and improve tolerance to the therapy.

In the case of positive airway pressure (PAP) therapy, a PAP apparatus (that includes a blower and a humidifier) can be used to provide pressure therapy to the patient or user. This therapy may be in the form of, for example, continuous positive airway pressure therapy (CPAP). As with noninvasive ventilation and high flow therapy, humidifying the gases inspired during PAP therapy can mitigate drying, increase comfort, and improve tolerance.

In the case of surgical gases, or invasive ventilation (when the gases delivered to the patient bypass the upper airway), humidification of the gases has been found to improve patient comfort and provide physiological benefits (such as improved mucus transport). Furthermore, humidification can be necessary for improving post-operative outcomes, and for ensuring patient or user safety. For example, humidification can help to prevent airway obstruction (caused by inspissation of airway secretion) and/or disruption of the airway epithelium.

In any of the therapies mentioned above, humidified gases can be delivered to a patient via a gas supply tube. This tube can be heated by an in-built tube heating element. Heating the gas supply tube prevents condensation of the warm, humid breathing gases as they travel from the humidifier to the patient. Preventing this condensation helps to ensure that the breathing gases are supplied to the patient or user at the required temperature and humidity. EP2775277 describes a wire heated tube with a temperature control system for a humidifier for a respiratory apparatus.

### SUMMARY

If a humidifier malfunctions during use, then the gases supplied to the patient (via the gas supply tube) may not be correctly humidified. Incorrect humidification can result in risks to the patient (or other user). These risks can include, for example, transient currents in the tube heating element, prolonged exposure of the airway to dry gases, discomfort, condensation blocking the airflow, high enthalpy being conveyed to the patient, high surface temperature of the gas supply tube or damage to the respiratory humidifier system.

For a humidifier to operate correctly, all of the sub-systems within it must operate correctly. If a fault (capable of causing malfunction) occurs in any of these sub-systems, the humidifier must have means to identify the fault and alert the person administering the therapy (e.g. a healthcare professional or the patient themselves in the case of home-based administration). The present disclosure is supply tube heating element and the power supply path-which transfers power from the heater base unit to the gas supply tube heating element.

Some existing humidifiers have systems that can detect fault conditions (in the gas supply tube heating element and the power supply path). However, these systems have limitations. For example, these fault-detection systems may be complex, may perform only a limited range of tests, may not allow detection of certain faults during therapy, and may only be capable of detecting faults when a gas supply tube with a heating element is connected.

The present disclosure provides examples of humidifier fault-detection systems that overcome some of the limitations in existing humidifier fault-detection systems - specifically those used to detect faults associated with the gas supply tube heating element and its power supply path. The fault-detection systems and methods in the present disclosure are based on monitoring power attributes when a switchable load is switched into the power supply path. This switching and monitoring may be performed regardless of whether there is a gas supply tube (with a functioning heating element) attached. This switching and monitoring may also be performed during therapy without any interruption to therapy. The fault-detection systems disclosed herein can also have any of the following advantages, and/or other advantages. For example, the monitoring systems and methods disclosed herein allow a wide range of tests to be performed using relatively simple additional components. Such tests may include detecting any of a number of possible fault conditions. These fault conditions may include a power flow controller fault (such as an open-circuit or short-circuit fault), a current sensor fault, a tube heating element fault, or a general fault. Such tests may also be used to detect the presence of a tube heating element. Furthermore, the implementation is simple, reliable, robust and inexpensive.

The detection and/or alarm methods described herein can be incorporated into a variety of respiratory and/or surgical humidifier systems, such as CPAP devices, high flow therapy devices, surgical humidifiers, respiratory humidifiers, infant CPAP devices, infant high flow devices, NIV therapy devices, and the like.

In some configurations, a respiratory or surgical humidifier can comprise power supply lines configured to supply power via a supply path to respective output terminals; a switchable load electrically connected across the output terminals; a control circuit configured to control switching of the switchable load; and a sensor that monitors at least one attribute of power supplied via the supply path and provides attribute information to the control circuit. The control circuit may be configured to control the switchable load to operate in different states and determine operation of the respiratory or surgical humidifier based on the attribute information for the different states.

In some configurations, the control circuit can be configured to control the switchable load to test the respiratory or surgical humidifier whilst power is supplied to a tube heating element electrically connected across the output terminals.

In some configurations, the respiratory or surgical humidifier can include a power flow controller controlled by the control circuit to vary the level of power supplied via the supply path.

In some configurations, the power flow controller can be a controllable switch in the supply path.

In some configurations, the control circuit can control state combinations of the switchable load and power flow controller to monitor operation of the respiratory or surgical humidifier based on the attribute information for the different state combinations.

In some configurations, the switchable load can include a controllable switch and an impedance.

In some configurations, the controllable switch can be a semiconductor switch.

In some configurations, the impedance can be a resistor.

In some configurations, the impedance can include a reactive impedance.

In some configurations, a voltage sensor can be provided across the power supply lines.

In some configurations, the attribute information can include the voltage across the power supply lines.

In some configurations, the sensor can be a current sensor.

In some configurations, the sensor can be an inductive, Hall effect, or shunt resistance-based sensor.

In some configurations, the attribute information includes the current flowing in the supply path.

In some configurations, the control circuit can be configured to switch the power flow controller on whilst the switchable load is switched off and receive current information from the current sensor representing a first current level.

In some configurations, the control circuit can be configured to detect an error if the first current level is outside a permitted range.

In some configurations, the control circuit can be configured to switch the power flow controller on whilst the switchable load is switched on and receive current information from the current sensor representing a second current level.

In some configurations, the control circuit can be configured to detect an error if the difference between the first and second current levels is outside a permitted range.

In some configurations, the control circuit can be configured to detect an error if the difference between the first current level and an expected first current level or the difference between the second current level and an expected second current level is outside a permitted range.

In some configurations, the expected first current level, expected second current level or the permitted range for the difference between the first and second current levels can be determined on the basis of a voltage level across the output terminals and a switchable load impedance and a tube heating element impedance.

In some configurations, the permitted current range can be a permitted current level plus or minus a margin of error.

In some configurations, the control circuit can be configured to detect a fault if neither the first or second current is above a minimum threshold level.

In some configurations, the control circuit can be configured to receive current information from the current sensor when the power flow controller is switched off and the switchable load is switched on, and detect a fault if the current is above a low current threshold level.

In some configurations, a transient current detector can be provided in the supply path.

In some configurations, the transient current detector can include a pair of transistors configured to detect positive or negative transient voltages across an inductor in the supply path.

In some configurations, a respiratory or surgical humidifier can comprise a power flow controller configured to control power supplied via a supply path to respective output terminals; a switchable load electrically connected across the output terminals; a current sensor that monitors current supplied via the supply path to develop current information; a control circuit configured to receive the current information from the current sensor, control the power flow controller to supply power via the output terminals, and control switching of the switchable load with the control circuit configured to: switch the power flow controller on whilst the switchable load is switched off and receive current information from the current sensor representing a first current level; switch the power flow controller on whilst the switchable load is switched on and receive current information from the current sensor representing a second current level; and determine operation of the respiratory humidifier based on at least the first and second current levels.

In some configurations, a respiratory or surgical humidifier can comprise power supply lines configured to supply power via a supply path to respective output terminals; a switchable load electrically connected across the output terminals; a current sensor that monitors current supplied via the supply path to develop current information; a control circuit configured to receive the current information from the current sensor and control switching of the switchable load in which the control circuit is configured to switch the switchable load off and receive current information from the current sensor representing a first current level; switch the switchable load on and receive current information from the current sensor representing a second current level; and detect an error if the difference between the first and second current levels is outside a permitted range.

In some configurations, a respiratory or surgical humidifier can comprise a power flow controller configured to supply power via a supply path to respective output terminals; a switchable load electrically connected across the output terminals; a current sensor that monitors current supplied via the supply path to develop current information; a control circuit configured to receive the current information from the current sensor, control switching of the power flow controller to supply power via the output terminals and control switching of the switchable load with the control circuit configured to: switch the power flow controller on whilst the switchable load is switched off and receive current information from the current sensor representing a first current level; switch the power flow controller on whilst the switchable load is switched on and receive current information from the current sensor representing a second current level; detect an error if the second current level is outside a permitted range; and detect an error if the difference between the first and second current levels is outside a permitted range.

In some configurations, a respiratory or surgical humidifier can comprise: a power flow controller configured to supply power via a supply path to respective output terminals; a switchable load electrically connected across the output terminals; a current sensor that monitors current supplied via the supply path to develop current information; a control circuit configured to receive the current information from the current sensor, control switching of the power flow controller to supply power via the output terminals, and control switching of the switchable load with the control circuit configured to: switch the power flow controller on whilst the switchable load is switched on and receive current information from the current sensor representing a test current level; and detect an error if the test current level is outside a permitted range.

In some configurations, a respiratory or surgical humidifier can comprise a power flow controller configured to control power supplied via a supply path to respective output terminals; a switchable load electrically connected across the output terminals; and a control circuit configured to control the switchable load whilst the power flow controller supplies power to the output terminals to perform in use monitoring of the respiratory or surgical humidifier.

In some configurations a respiratory or surgical humidifier can include a housing configured to receive a humidifier chamber; a heater plate configured to transfer heat to a humidifier chamber when received by the housing; a heater element configured to heat the heater plate; and a power controller configured to control a level of power supplied to the heater element.

In some configurations, a method of monitoring the operation of a respiratory or surgical humidifier having output terminals for supplying power to a heater element of a gas supply tube can comprise temporarily connecting a dummy load across the output terminals whilst power is supplied to the heater element and evaluating one or more power supply attributes to monitor correct operation of the respiratory humidifier.

In some configurations, an error in the operation of the respiratory or surgical humidifier can be detected if a current supplied via the output terminals is below a threshold value.

In some configurations a first current supplied via the output terminals can be measured when the dummy load is not connected across the output terminals and a second current supplied via the output terminals can be measured when the dummy load is electrically connected across the output terminals and an error in the operation of the respiratory or surgical humidifier can be detected if the difference between the first and second currents is outside a permitted range.

In some configurations a respiratory or surgical humidifier includes a transient current detector in the power supply path configured to detect positive or negative transient voltages across an inductor in the supply path.

### BRIEF DESCRIPTION OF THE DRAWINGS

These and other features, aspects, and advantages of the present disclosure are described with reference to the drawings of certain embodiments, which are intended to schematically illustrate certain embodiments and not to limit the disclosure.
Figure 1A illustrates schematically an example respiratory humidifier system.
Figure 1B illustrates schematically an example heater base unit of the respiratory humidifier system of Figure 1A.
Figure 1C illustrates schematically an example respiratory humidifier system.
Figure 1D illustrates schematically an example heater base unit of the respiratory humidifier system of Figure 1C.
Figure 1E illustrates schematically a partial view of the heater base unit and an example breathing circuit tube heating element adapter of Figure 1C.
Figure 2 illustrates an example circuit for monitoring the operation of a respiratory or surgical humidifier.
Figure 3 illustrates a modified example circuit for monitoring the operation of a respiratory or surgical humidifier.
Figure 4 illustrates an example circuit (including a transient current detector) for monitoring the operation of a respiratory or surgical humidifier.
Figure 5 illustrates an example circuit for the transient current detector circuit shown in Figure 3.
Figure 6 illustrates a modified form of the transient current detector circuit shown in Figure 5.

### DETAILED DESCRIPTION

Although certain embodiments and examples are described below, those of skill in the art will appreciate that the disclosure extends beyond the specifically disclosed embodiments and/or uses and obvious modifications and equivalents thereof. Thus, it is intended that the scope of the disclosure herein disclosed should not be limited by any particular embodiments described below. For example, component values and operating parameters are examples only and are not limiting.

### Example Respiratory or Surgical Humidifier

The present disclosure provides examples of a respiratory humidifier configured to supply humidified and/or heated gas to a patient or user in multiple modes. The modes for the respiratory humidifier can include at least an invasive mode (for example, for patients with a bypassed airway) and a noninvasive mode (for example, for patients or users with breathing masks). Each mode can have individualized humidity output, which can be expressed as dew point output set points. For example, a user can select a set point appropriate for the current mode of operation. The non-invasive mode can have selectable dew point set points of, for example, 31 degrees Celsius, 29 degrees Celsius, 27 degrees Celsius, or others. The invasive mode can have a dew point set point of 37 degrees Celsius or others. Alternatively, the dew point set points may be continuously variable between upper and lower limits. Some respiratory humidifier systems disclosed herein can also include a high flow, unsealed mode or any other modes known to those of skill in the art. The disclosed circuits and methods may be similarly applied in a surgical humidifier which may be used, for example, in laparoscopic surgery.

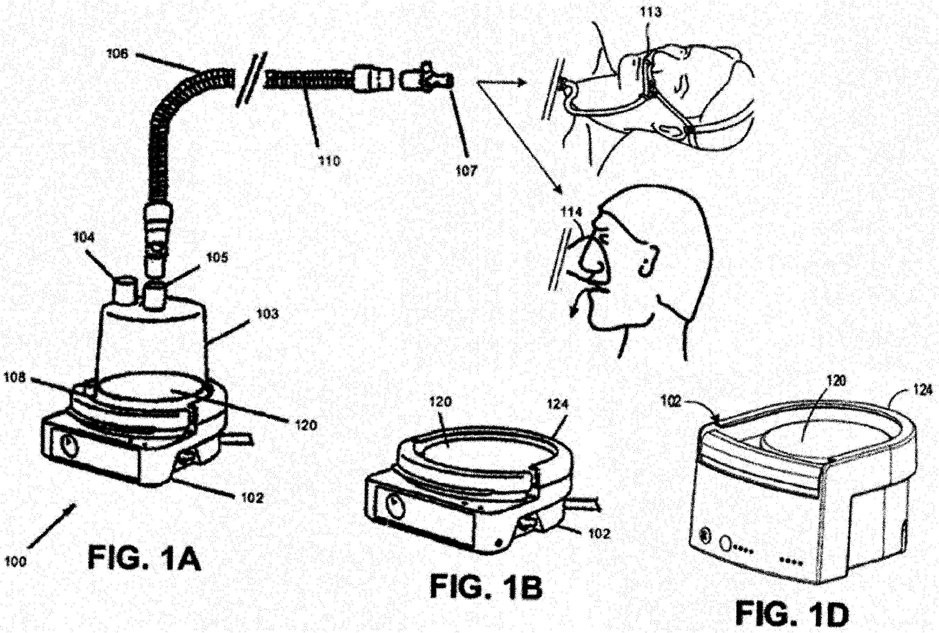

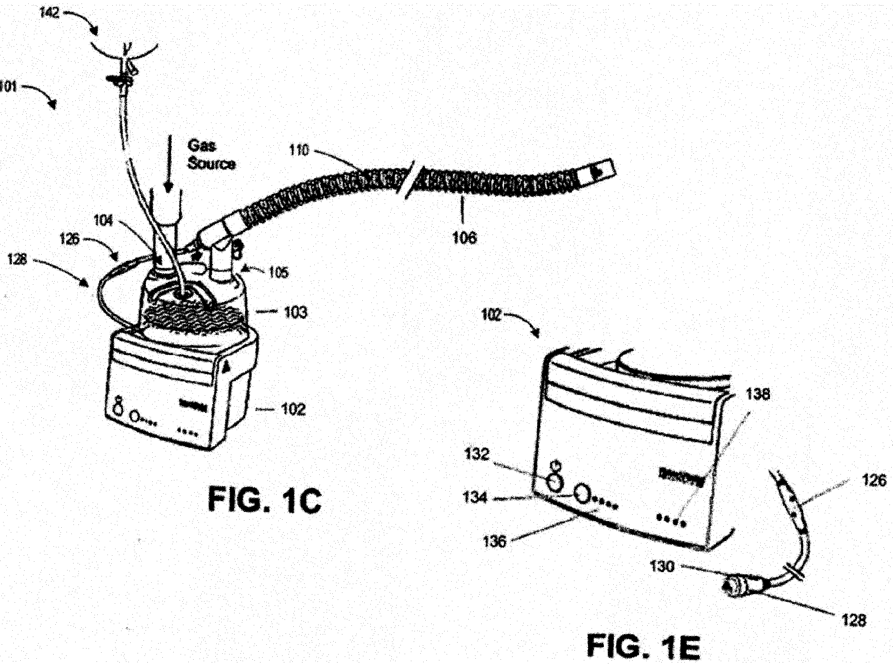

Referring to Figures 1A and 1C, an example respiratory humidifying system 100, 101 can include a heater base unit 102 having a heater plate 120 (see Figures 1B and 1D). The heater plate 120 can include one or more heating element(s). The heater base unit 102 can have a housing and a controller (for example, a microprocessor) contained within the housing for controlling the supply of energy to the heating element(s) of the heater plate 120.

The humidifier heater plate 120 can have a temperature sensor (for example, a temperature transducer, thermistor, or other types of temperature sensor). Multiple different temperature sensors may also be used. The temperature sensor can measure a temperature of the heater plate 120. The temperature sensor can be in electrical communication with the controller in the heater base unit 102 so that the controller can monitor the temperature of the heater plate 120.

The humidifier chamber 103 can be removably received and retained on the heater base unit 102, such that the humidifier chamber base is positioned in contact with the heater plate 120 in the heater base unit 102. Referring to Figures 1B and 1D, which illustrate examples of the heater base unit 102 of Figures 1A and 1C respectively, the humidifying base 102 can have a collar 124 for engaging with a flange on the humidifier chamber 103, such as shown in Figures 1A and 1C. The collar 124 defines a lip that engages a flange of the humidifier chamber 103 to retain the humidifier chamber 103 in an operative position on the heater base 102. The humidifier chamber 103 can include a thermally conductive base. When engaged with the heater base unit 102, the conductive base of the humidifier chamber 103 can be in contact with the heater plate 120, such as an upper surface of the heater plate 120. Water inside the chamber 103 is heated when a power signal is sent to the heating element to energize the heating element. The chamber 103 can also be connected to a water source 142 (Figure 1C), which can add water to the chamber 103 when the water is low or completely out in the chamber 103. Adding of water can be manually performed, such as upon a warning from the system 101 that there may be a low water or water-out condition, or automatically performed, such as using a float valve connected to a water supply.

With continued reference to Figures 1A and 1C, the gases to be humidified can include one or more of air, oxygen, anesthetic, other auxiliary gases, or any mixture of gases. The gases can be supplied to the humidifier chamber 103 through a gas inlet 104, which can be connected to a gas source, such as a ventilator, in the case of CPAP therapy a CPAP blower, or a remote source. For high flow therapy, a blower or further alternatively a wall source with a flow and/or pressure regulator can supply the gases. The humidifier chamber 103 also includes a gases outlet 105, which can connect to a breathing circuit 106. The breathing circuit 106 can convey humidified and heated gases to a patient or user. As shown in Figure 1A, a patient end 107 of the breathing circuit 106 can connect to a patient interface, such as a nasal cannula 113 or a nasal mask 114. The breathing circuit 106 can also connect to other types of patient or user interfaces, such as a full-face mask, total-face mask, nasal pillows mask, endotracheal tube, or others. The breathing circuit 106 of Figure 1C can similarly be connected to any suitable patient interface. The breathing circuit 106 may include a gas supply tube with or without a tube heating element. The respiratory humidifying system 100, 101 may be configured for use with both heated and unheated gas supply tubes or breathing circuits.

A tube heating element 110 (such as one or more heater wires embedded in the gas supply tube wall, contained within the gas supply tube, or wrapped around the outside of the gas supply tube) can be provided in the breathing circuit 106. The tube heating element 110 can help prevent condensation of the humidified gases within the breathing circuit 106. The tube heating element 110 can also optionally be in electrical communication with the controller in the heater base unit 102. As shown in Figures 1C and 1E, a breathing circuit tube heating element adaptor cable 128 can have two connectors at two ends of the cable 128 for coupling the tube heating element 110 to the heater base unit 102 (such as to the controller of the heater base unit 102). Alternatively, the tube heating element adapter cable may be permanently electrically connected to a tube heating element supply circuit of the heater base unit 102, for example by soldering. The tube heating element adaptor cable 128 can facilitate an easy connection between the tube heating element 110 and the heater base unit 102. The tube heating element 110 is controlled by the controller, including the controlling of power to the tube heating element 110. The tube heating element 110 in the breathing circuit 106 reduces condensation and ensures the temperature and/or humidity of gases is maintained in a predetermined range. The tube heating element adaptor cable 128 can also include an ambient temperature sensor 126, which can allow the system 101 to adjust the tube heating element 110 power and/or heater plate power to compensate for ambient temperatures or changes in the ambient temperature. The ambient temperature sensor can alternatively be located anywhere that is exposed to the ambient air. A tube heating element indicator 130 can be embedded into the connector that couples to the heater base unit 102. The tube heating element indicator 130 can be illuminated when a properly functioning tube heating element 110 is connected to the heater base unit 102, and the system 101 can heat the gas inside the breathing circuit 106 via the tube heating element 110 to minimize condensate in addition to heating the gas passing through the humidifier chamber 103 via the heater plate 120. If the tube heating element 110 is malfunctioning or not connected, the tube heating element indicator 130 is not illuminated (or is flashing), and the system 101 may heat the gas only by heating the water in the chamber 103 via the heater plate 120. Alternatively, the tube heating element indicator 130 may be illuminated when there is a fault or a disconnection of the adaptor cable 128 from the tube heating element 110. The illuminated indicator 130 can act as a visual message or a visual warning. The indicator 130 may not be illuminated if the tube heating element 110 is functioning correctly.

The controller of the respiratory humidifier system 100, 101 can control at least the heater plate 120, and preferably or optionally also the tube heating element 110, without additional sensors (for example, in the humidifier chamber, at the chamber outlet, in the breathing circuit, and/or elsewhere in the system). This can be achieved by estimating a heater plate operating point required to deliver a required humidity. For a given respiratory humidifier system, the controller can determine an appropriate level of power to apply to the heater plate 120. Applying power to the heater 120 can generate humidity and heat the gases. The heater plate power and temperature can be controlled to generate a predetermined amount of humidity. Additionally, the parameters can also optionally be used by the controller to provide a more appropriate level of energization to the tube heating element 110. As shown in Figures 1C and 1E, the system 101 can also include the ambient temperature sensor 126. The ambient temperature sensor can be located anywhere that is exposed to the ambient air. For example, the system 101 can include the ambient temperature sensor 126 on the tube heating element adaptor cable 128.

As shown in Figure 1E, a front panel of the heater base unit 102 can include a plurality of user controls and indicators, such as a power button 132, a humidity setting push button 134, and a plurality of (such as three, four, five or more) humidity settings indicators 136 (which can include LED lights) next to the humidity setting push button 134. The locations, shapes, and sizes of the user controls and indicators are not limiting. There can be four levels of humidity settings available which are indicated by the four humidity setting indicators 136. The four humidity settings may correspond to different types of therapies provided to a patient. For example, the highest amount of humidity can be selected when the humidifier is operating in an invasive therapy mode. The lowest amount of humidity may be applied in a low flow oxygen therapy mode. The amount of humidity can be selected based on therapeutic requirements or therapy type, or it may be predefined. Alternatively, the humidifier 100, 101 may include a controller that is configured to automatically select the amount of humidity to be delivered based on a therapy mode, the patient, or the type of therapy being applied to the patient. Optionally, the humidifier 100, 101 may include a display or touch screen that may communicate information to the user. The touch screen may also be configured to receive inputs from the user.

The humidity level can be adjusted by pressing the humidity settings push button 134, which can also be a momentary push button. The front panel can also include a plurality of alarm indicators 138 (which can include LED lights) to indicate the following non-limiting examples of conditions: "water out" condition (including low water and water-out), tube heating element adaptor not connected, audible alarm muted, and a "See Manual" indication used to indicate that a fault has occurred within the system 101.

The system 101 can be suitable for providing respiratory therapy for different purposes, such as for critical care (for example, in the hospital) and home care. The system 101 is suitable for providing invasive, non-invasive and high flow therapies for both adult and pediatric patients.

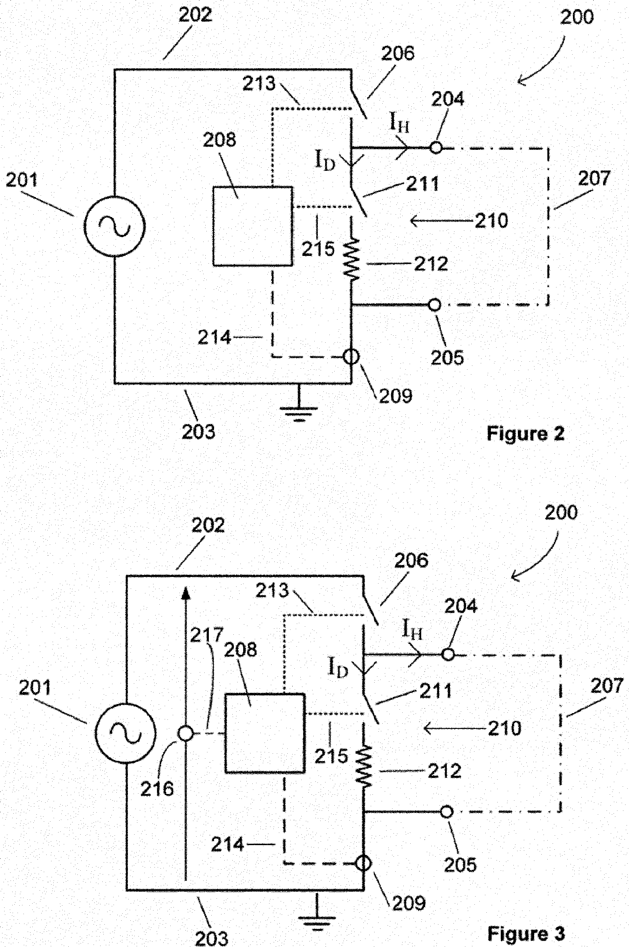

With reference to Figure 2, an example tube heating element supply circuit for a respiratory or surgical humidifier can include an electric power source 201 that can supply power via a power supply path (which includes a power flow controller 206 and power supply lines 202 and 203) to output terminals 204 and 205. Power source 201 could be an AC or DC power supply but in this example is an AC power supply (including a transformer that reduces the supply voltage from mains voltage to about 22 Volts). A tube heating element 207 can be connected to output terminals 204 and 205 when the respiratory or surgical humidifier is providing therapy to heat gases within a gas supply tube. The tube heating element 207 can be in the form of one or more heater wires embedded in a gas supply tube wall, contained within the gas supply tube, wrapped around the outside of the gas supply tube, or otherwise.

In this example, power flow controller 206 can be a controllable switch, such as a semiconductor switch, controlled by a control circuit 208 via control line 213. Power flow controller 206 could alternatively supply a number of different power levels or continuously vary the level of power supplied via the supply path. Control circuit 208 can include a microprocessor. Control circuit 208 can control the switching of flow controller 206 when a tube heating element is connected to ensure that a required amount of heating is provided to a gas within a gas supply tube. The tube heating element supply circuit can include a current sensor 209 for monitoring the current flowing in the supply path and providing attribute information in the form of the current level via data line 214 to control circuit 208. The current sensor 209 can be an inductive, Hall effect or shunt resistor-based sensor.

The tube heating element supply circuit can also include a switchable load 210 electrically connected across the output terminals 204 and 205. Switchable load 210 can include a controllable switch 211 in series with a "dummy" load 212. The controllable switch can be a semiconductor switch controlled by control circuit 208 via control line 215. The load 212 can be a resistance but can also be another form of impedance or composite impedance.

The control circuit 208 can be configured to control the switchable load 210 to test the operation of the respiratory or surgical humidifier either when no tube heating element 207 is connected across output terminals 204 and 205 or whilst power is supplied to the output terminals with a tube heating element 207 connected across output terminals 204 and 205 during therapy.

The control circuit 208 can also be configured to control the power flow controller 206 and the switchable load 210 to operate in different state combinations and monitor the operation of the respiratory or surgical humidifier. This monitoring may be based on the attribute information received from the current sensor 209 for different switch states of the switchable load 210 and power flow controller 206.

In one example the tube heating element supply circuit 200 of Figure 2 may be used in a fault checking method (where a tube heating element 207 is connected across terminals 204 and 205) where the following steps can be performed:
i. Control circuit 208 controls power flow controller 206 to switch on (if it is open at the start of the fault check) whilst switch 211 of switchable load 210 is switched off.
ii. Control circuit 208 receives attribute information in the form of a current level measurement from current sensor 209. In this case, where the power flow controller 206 and switch 211 are working as intended, a first current level (I_{H}) flowing through tube heating element 207 is measured.
iii. Control circuit 208 controls switch 211 of controllable load 210 to turn on.
iv. Control circuit 208 receives attribute information in the form of a second current level measurement from current sensor 209. In this case, where the power flow controller 206 and switch 211 are working as intended, the second current level measured is the sum of I_{H} flowing through tube heating element 207 and the current I_{D} flowing through the dummy load 212 (in this case a resistor).
v. Control circuit 208 (in this case including a microprocessor) determines the difference between the currents (referred to as "ΔI") being the second current level measured in step iv. minus the first current level measured in step ii.
vi. Control circuit 208 can determine whether there is a fault (potentially a fault in current sensor 209 or alternatively a fault in dummy load 212, dummy load switch 211 or power flow controller 206) by checking whether the difference in current (ΔI) is within a permitted current range.
vii. Control circuit 208 can determine whether a tube heating element is present or whether there is a fault (potentially a fault in the power flow controller 206 or tube heating element 207) by checking whether the first current level measured in step ii., (I_{H}), is within a permitted range.
viii. Control circuit 208 can also determine that there is a fault in power flow controller 206 if the current measured by current sensor 209 is above a low current threshold level (at or near zero) when power flow controller 206 is switched off and dummy load switch 211 is switched on. With dummy switch 211 switched on such a fault may be detected whether a tube heating element is in circuit or not. Alternatively, where for example a heater wire is permanently connected, just the current I_{H} may be measured when power flow controller 206 is switched off and dummy load switch 211 is switched off. If the current is above a low current threshold level, for example 0.1 A, it may be determined that there is a fault in power flow controller 206.

It will be appreciated that not all of above steps need be carried out and that in some implementations only one or a selection of steps may be implemented. It will also be appreciated that the selected steps may be carried out in a different sequence.

By using the difference between currents in step vi. the fault checking method above may be performed whether a tube heating element is present or not, whether a tube heating element (if present) is functioning correctly or not, and whether a connected tube heating element is being used to provide therapy or not, as will be explained below. As the current difference may be expressed as:$ΔI = \left({I}_{D}+{I}_{H}\right) - {I}_{H}$ Therefore: I_{D}

Thus, the current ΔI will be equal to I_{D} irrespective of whether a heater wire is present or absent. This is because the current flowing through the heater wire is cancelled out in all cases.

Knowing that the test in step vi. isolates the current component I_{D} flowing through the controllable load 210, the expected current sensed by current sensor 209 will be the supply voltage divided by the resistance of load 212. The permitted current range in step vi. may be the expected current plus or minus a permitted margin of error. For example, assuming no fault is present, with a 22V supply voltage and a 470 Ω resistance, the permitted range may be 22/470 +/- 20% (or some other acceptable error range). The test in step vi. may therefore test whether the control circuit is operating outside a permitted range (potentially due to a fault in the current sensor or a component in the supply path).

In step vii. the current I_{H} flowing through the tube heating element 207 may be monitored to determine whether a tube heating element 207 is present or not and, if present, if it is operating within an acceptable range (assuming no other faults have been identified in step vi.). The permitted current range may be the expected current plus or minus a permitted margin of error (for example +/- 20%). In this case the expected current may be I_{H} = V/R_{Heating_Element_207} where V is the supply voltage across the output terminals. Alternatively, the expected current could be based on the current measured at step iv.. In this case the expected current would be I_{D} + I_{H} = V/R_{Resistor_212} + V/R_{Heating_Element_207} and this may be tested against the current measured in step iv. For example, with a 22V supply voltage, a 470 Ω resistance 212, and a 24 Ω heater wire, the permitted range may be (22/470 + 22/24) +/- 20% (or some other acceptable error range). The test can alternatively simply be whether the expected current is below a lower threshold (i.e. I_{D} + I_{H} < (V/R_{Resistor_212} + V/R_{Heating_Element_207}) * x%, where x provides a margin of error), or, using the values above, where the expected current is less than 0.8 * (22/470 + 22/24).

Where no fault is detected in step vi. and the current I_{H} is zero or negligible control circuit 208 may determine that a tube heating element is not present or not operating correctly. In some configurations a tube heating element may only be required where high levels of humidification are required. In such configurations if a user attempts to use a high humidification level where a tube heating element is not present or not working correctly control circuit 208 may provide a visual alert via indicators 136 and/or provide an audible alert and/or communicate an alert condition to a remote device and/or inhibit certain functionality (depending upon the nature of the fault detected). For lower levels of humidification, therapy may be provided without the need for the tube heating element to be driven.

Control circuit 208 can run a fault check when the humidifier starts up and then intermittently during operation (e.g., every 10 minutes or every 2 hours). Each fault check occurrence has a negligible effect on the operation of the humidifier, due to the independence of the controllable load 210 from the tube heating element current path.

With reference to Figure 3, an example tube heating element supply circuit for a respiratory or surgical humidifier can also include a voltage sensor 216 for sensing the voltage across the voltage source and providing voltage information via a data line 217. The remaining circuit components are as in Figure 2 and so the same numerals have been used. Operation may be the same as for Figure 2 except that instead of assuming a constant voltage supply the actual supply voltage may be used by control circuit 208. This circuit may be appropriate where there is substantial variation of the supply voltage. This circuit also allows the identification of a fault due to the supply voltage not being within a required range.

In one example, the tube heating element supply circuit of Figure 3 can perform a fault checking method (where a tube heating element 207 is connected across terminals 204 and 205) where the following steps are performed:
i. Control circuit 208 controls power flow controller 206 to switch it on (if it is open at the start of the fault check) whilst switch 211 of switchable load 210 is switched off.
ii. Control circuit 208 receives attribute information in the form of a first current level measurement from current sensor 209. In this case the current I_{H} flowing through tube heating element 207 is measured. Control circuit 208 also receives attribute information in the form of a voltage level measurement from voltage sensor 216. A resistance can then be calculated using Ohm's law representing the resistance R_{H} of the tube heating element 207.
iii. Control circuit 208 controls switch 211 of controllable load 210 to turn on.
iv. Control circuit 208 receives attribute information in the form of a second current level measurement from current sensor 209. In this case the sum of current I_{H} flowing through tube heating element 207 and the current I_{D} flowing through resistor 212 is measured (I_{D} + I_{H}). Control circuit 208 also receives attribute information in the form of a voltage level (Vᵢᵥ) measurement from voltage sensor 216.
v. Control circuit 208 (in this case including a microprocessor) determines an expected second current level Iₑₓₚ according to the formula: ${I}_{exp} = \left(\frac{{V}_{iv}}{{R}_{H}}+\frac{{V}_{iv}}{R}\right)$ where R is the known resistance 212.
vi. Control circuit 208 determines the difference between the current levels (εI) being the expected second current level Iₑₓₚ minus the first current level (I_{H}) measured in step ii.
vii. Control circuit 208 determines whether there is a fault in current sensor 209 (or another component in the supply path) by checking whether the difference in current (εI) is within a permitted current range.
viii. Control circuit 208 can also determine whether there is a fault in the power flow controller 206 by checking whether the second current level measured in step iv. (I_{D} + I_{H}) is within a permitted range. Control circuit 208 can determine whether a tube heating element is present or whether there is a fault (potentially a fault in the power flow controller 206 or tube heating element 207) by checking whether the first current level (I_{H}) measured in step ii. is within a permitted variance from an expected first current level Vᵢᵥ/R_{H}.
ix. Control circuit 208 can also determine whether there is a fault in power flow controller 206 by checking whether the second current level (I_{D} + I_{H}) is above a low current threshold level (at or near zero) when power flow controller 206 is switched off and dummy load switch 211 is switched on. Alternatively, where for example a heater wire is permanently connected, just the first current level (I_{H}) may be measured when power flow controller 206 is switched off and dummy load switch 211 is switched off. If the current is above a low current threshold level, for example 0.1 A, it may be determined that there is a fault in power flow controller 206.

Should it be determined that a fault condition exists, control circuit 208 can turn off the power flow controller 206 and/or provide a visual alert via indicators 136 and/or provide an audible alert and/or communicate an alert condition to a remote device and/or inhibit certain functionality (depending upon the nature of the fault detected).

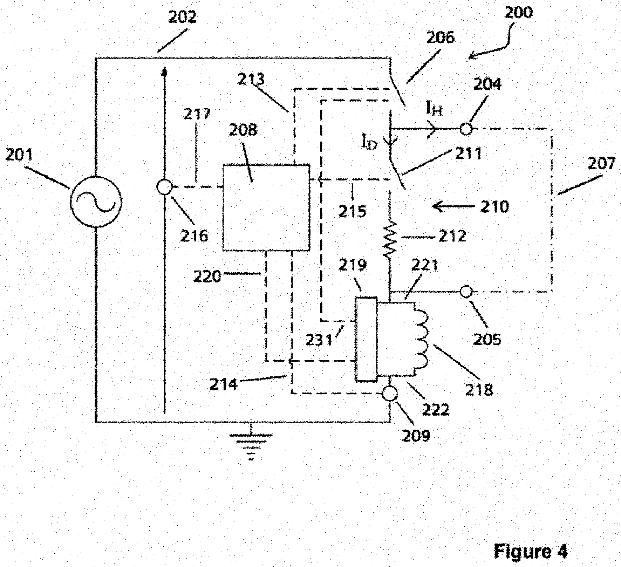

With reference to Figure 4, an example tube heating element supply circuit for a respiratory or surgical humidifier can also include a transient current detector capable of detecting transient currents outside of a permitted range. Transient currents in the form of short-lived, aperiodic spikes in the supply current could potentially arise in the case of a heater wire breakage and could potentially cause a gas supply tube to have a high surface temperature, melt or ignite from sparking. These situations can also occur from EMI and/or mains surge. To protect against these situations, a transient current detector can cause power supply to a tube heating element to be stopped where transient currents outside a permitted range are detected.

In the circuit shown in Figure 4, like elements to those shown in Figures 2 and 3 are given like numbers. As shown in Figure 4, an inductor 218 may be included in the power supply path with sense lines 221 and 222 connected to a transient current detection circuit 219. When transient current detection circuit 219 detects a transient current outside of a permitted range it can send a transient current detection signal or a feedback signal via signal line 220 to control circuit 208. In response, control circuit 208 can turn power flow controller 206 off to terminate power supply to the tube heating element. Additionally and/or alternatively the transient current detection circuit 219 can turn power flow controller 206 off directly via signal line 231 to terminate power supply to the tube heating element. Additionally and/or alternatively control circuit 208 can turn off other components, such as the heater plate, or generate an alarm when transient current detection circuit 219 detects a transient current outside of a permitted range Although Figure 4 illustrates a circuit further comprising the switchable load 210 described above, it will be appreciated that the disclosed transient current detector may alternatively be used independently of the disclosed fault detection methods and circuits.

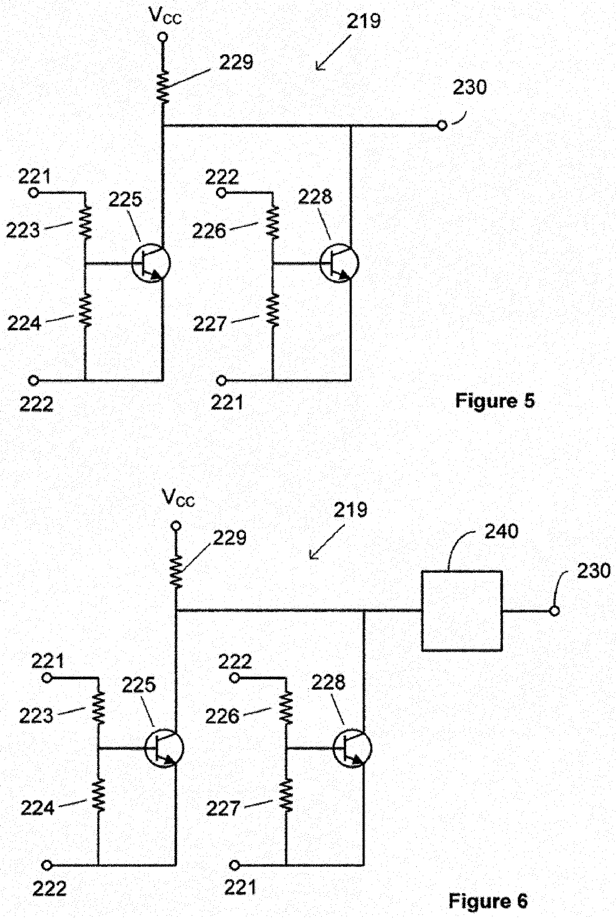

Figure 5 shows a simple transient current detection circuit 219 that can be used to detect transient currents. A first transistor detection circuit, including transistor 225 and biasing resistors 223 and 224, is connected to sense lines 221 and 222 and switches on when a difference in sense line voltages (V₂₂₁ - V₂₂₂) is above a permitted level. A second transistor detection circuit, including transistor 228 and biasing resistors 226 and 227, is also connected to sense lines 221 and 222 and switches on when a difference in sense line voltages (V₂₂₂ - V₂₂₁) is above a permitted level. Biasing resistor 229 holds transient current detection output signal line 230 high when both transistor 225 and 228 are switched off (high resistance). Output signal line 230 is connected to power flow controller 206 by line 220. When either transistor 225 or 228 switches on, transient current detection output signal line 230 is pulled down and in response control circuit 208 can turn off power flow controller 206. Figure 6 shows a modified transient current detection circuit 219 which is the same as Figure 5 except that a signal conditioning circuit 240 is provided prior to output signal line 230. It may be desirable for output signal line 230 to be held or extended in a pulled down state for a period of time rather than just during the period that transient currents are detected. The signal conditioning circuit 240 can be a monostable multivibrator with a pre-defined delay or some other suitable circuit.

By connecting sense lines 221 and 222 with opposite polarity to the transistor detection circuits a window detector may be provided to detect positive and negative transient currents using a simple circuit employing only two transistors without using a bridge rectifier or a negative power supply.

Methods and processes described herein may be embodied in, and partially or fully automated via, software code modules executed by one or more general and/or special purpose computers. The word "module" refers to logic embodied in hardware and/or firmware, or to a collection of software instructions, possibly having entry and exit points, written in a programming language, such as, for example, C or C++. A software module may be compiled and linked into an executable program, installed in a dynamically linked library, or may be written in an interpreted programming language such as, for example, BASIC, Perl, or Python. It will be appreciated that software modules may be callable from other modules or from themselves, and/or may be invoked in response to detected events or interrupts. Software instructions may be embedded in non-volatile memory, such as an erasable programmable read-only memory (EPROM). It will be further appreciated that hardware modules may comprise connected logic units, such as gates, flip-flops and/or application specific integrated circuits, and/or may comprise programmable units, such as programmable gate arrays and/or processors. The modules described herein can be implemented as software modules, but also may be represented in hardware and/or firmware. Moreover, although in some embodiments a module may be separately compiled, in other embodiments a module may represent a subset of instructions of a separately compiled program and may not have an interface available to other logical program units.

In certain embodiments, code modules may be implemented and/or stored in any type of computer-readable medium or other computer storage device. In some systems, data (and/or metadata) input to the system, data generated by the system, and/or data used by the system can be stored in any type of computer data repository, such as a relational database and/or flat file system. Any of the systems, methods, and processes described herein may include an interface configured to permit interaction with users, operators, other systems, components, programs, and so forth.

It should be emphasized that many variations and modifications may be made to the embodiments described herein, the elements of which are to be understood as being among other acceptable examples. All such modifications and variations are intended to be included herein within the scope of this disclosure and protected by the following claims. Further, nothing in the foregoing disclosure is intended to imply that any particular component, characteristic or process step is necessary or essential.

## Claims

1. A respiratory or surgical humidifier comprising:
a. output terminals (204, 205) configured such that a tube heating element can be connected across the output terminals;
b. power supply lines (202, 203) configured to supply power via a supply path to the output terminals (204, 205);
c. a switchable load (210);
d. a control circuit (208) configured to control switching of the switchable load (210); and
e. a sensor (209; 209, 216);
**characterized in that**:
the switchable load is electrically connected across the output terminals;
the sensor monitors at least one attribute of power supplied via the supply path and provides attribute information to the control circuit; and
the control circuit is configured to control the switchable load to operate in different states and determine operation of the respiratory or surgical humidifier based on the attribute information for the different states.

2. The respiratory or surgical humidifier as claimed in claim 1 wherein the control circuit is configured to control the switchable load to test the respiratory or surgical humidifier whilst power is supplied to a tube heating element electrically connected across the output terminals.

3. The respiratory or surgical humidifier as claimed in claim 1 or claim 2 comprising a power flow controller controlled by the control circuit to vary the level of power supplied via the supply path, preferably wherein the power flow controller is a controllable switch in the supply path.

4. The respiratory or surgical humidifier as claimed in claim 3 wherein the control circuit controls state combinations of the switchable load and power flow controller to monitor operation of the respiratory or surgical humidifier based on the attribute information for the different state combinations.

5. The respiratory or surgical humidifier as claimed in any one of the preceding claims wherein the switchable load comprises:
a) a controllable switch, the controllable switch preferably comprising a semiconductor switch; and
b) an impedance, the impedance preferably comprising a resistor or a reactive impedance.

6. The respiratory or surgical humidifier as claimed in any one of the preceding claims comprising a voltage sensor across the power supply lines, preferably wherein the attribute information comprises the voltage across the power supply lines.

7. The respiratory or surgical humidifier as claimed in claim 3 or 4, or claim 5 or 6 when dependent on claim 3 or 4, wherein the sensor comprises a current sensor, preferably wherein the current sensor comprises an inductive, Hall effect or shunt resistance-based sensor, more preferably wherein the attribute information comprises a current flowing in the supply path.

8. The respiratory or surgical humidifier as claimed in claim 7 wherein the control circuit is configured to switch the power flow controller on whilst the switchable load is switched off and receive current information from the current sensor representing a first current level.

9. The respiratory or surgical humidifier as claimed in claim 8 wherein the control circuit is configured to detect an absence of a tube heating element or an error if the first current level is outside a permitted range.

10. The respiratory or surgical humidifier as claimed in claim 8 or claim 9 wherein the control circuit is configured to switch the power flow controller on whilst the switchable load is switched on and receive current information from the current sensor representing a second current level, preferably wherein:
the control circuit is configured to detect an error if the difference between the first and second current levels is outside a permitted range; or
the control circuit is configured to detect an error if the difference between the first current level and an expected first current level or the difference between the second current level and an expected second current level is outside a permitted range.

11. The respiratory or surgical humidifier as claimed in claim 10 wherein the expected first current level, expected second current level or the permitted range for the difference between the first and second current levels are determined on the basis of a voltage level across the output terminals and a switchable load impedance and a tube heating element impedance.

12. The respiratory or surgical humidifier as claimed in claim 10 or 11 wherein the permitted range for the difference between the first and second current levels or the difference between the first current level and the expected first current level or the difference between the second current level and the expected second current level is a permitted current level plus or minus a margin of error.

13. The respiratory or surgical humidifier as claimed in any one of claims 10 to 12 wherein the control circuit is configured to detect a fault if neither the first or second current level is above a minimum threshold level.

14. The respiratory or surgical humidifier as claimed in any one of claims 7 to 13 wherein the control circuit is configured to receive current information from the current sensor when the power flow controller is switched off and the switchable load is switched on, and detect a fault if the current is above a low current threshold level.

15. A method of monitoring the operation of a respiratory or surgical humidifier, the respiratory or surgical humidifier having output terminals (204, 205) for supplying power to a tube heating element of a humidifier supply tube when the tube heating element is connected across the output terminals (204, 205), **characterized in that**:
the method comprises temporarily connecting a dummy load across the output terminals whilst power is supplied to the tube heating element and evaluating one or more power supply attributes to monitor correct operation of the respiratory or surgical humidifier.

## Patentansprüche

1. Atmungs- oder Operationsbefeuchter, umfassend:
a. Ausgangsklemmen (204, 205), die so konfiguriert sind, dass ein Rohrheizelement über die Ausgangsklemmen angeschlossen werden kann;
b. Leistungsversorgungsleitungen (202, 203), die so konfiguriert sind, dass sie über einen Versorgungspfad Leistung zu den Ausgangsklemmen (204, 205) liefern;
c. eine schaltbare Last (210);
d. eine Steuerschaltung (208), die so konfiguriert ist, dass sie ein Schalten der schaltbaren Last (210) steuert; und
e. einen Sensor (209; 209, 216);
**dadurch gekennzeichnet, dass**:
die schaltbare Last elektrisch über die Ausgangsklemmen angeschlossen ist;
der Sensor mindestens ein Attribut der über den Versorgungspfad gelieferten Leistung überwacht und Attributinformationen an die Steuerschaltung bereitstellt; und
die Steuerschaltung so konfiguriert ist, dass sie die schaltbare Last steuert, in verschiedenen Zuständen zu arbeiten, und einen Betrieb des Atmungs- oder Operationsbefeuchters basierend auf den Attributinformationen für die verschiedenen Zustände bestimmt.

2. Atmungs- oder Operationsbefeuchter nach Anspruch 1, wobei die Steuerschaltung so konfiguriert ist, dass sie die schaltbare Last steuert, um den Atmungs- oder Operationsbefeuchter zu testen, während einem elektrisch über die Ausgangsklemmen angeschlossenen Rohrheizelement Leistung geliefert wird.

3. Atmungs- oder Operationsbefeuchter nach Anspruch 1 oder Anspruch 2, das eine Leistungsflusssteuereinheit, die von der Steuerschaltung gesteuert wird, umfasst, um den über den Versorgungspfad gelieferten Leistungspegel zu variieren, wobei die Leistungsflusssteuereinheit vorzugsweise ein steuerbarer Schalter im Versorgungspfad ist.

4. Atmungs- oder Operationsbefeuchter nach Anspruch 3, wobei die Steuerschaltung Zustandskombinationen der schaltbaren Last und der Leistungsflusssteuereinheit steuert, um einen Betrieb des Atmungs- oder Operationsbefeuchters basierend auf den Attributinformationen für die verschiedenen Zustandskombinationen zu überwachen.

5. Atmungs- oder Operationsbefeuchter nach einem der vorstehenden Ansprüche, wobei die schaltbare Last umfasst:
a) einen steuerbaren Schalter, wobei der steuerbare Schalter vorzugsweise einen Halbleiterschalter umfasst; und
b) eine Impedanz, wobei die Impedanz vorzugsweise einen Widerstand oder eine Blindimpedanz umfasst.

6. Atmungs- oder Operationsbefeuchter nach einem der vorstehenden Ansprüche, die einen Spannungssensor über die Leistungsversorgungsleitungen umfasst, wobei die Attributinformationen vorzugsweise die Spannung über die Leistungsversorgungsleitungen umfassen.

7. Atmungs- oder Operationsbefeuchter nach Anspruch 3 oder 4, oder Anspruch 5 oder 6, sofern er von Anspruch 3 oder 4 abhängt, wobei der Sensor einen Stromsensor umfasst, wobei der Stromsensor vorzugsweise einen induktiven, auf dem Hall-Effekt oder einem Shunt-Widerstand basierenden Sensor umfasst, wobei die Attributinformationen bevorzugter einen auf dem Strompfad fließenden Strom umfassen.

8. Atmungs- oder Operationsbefeuchter nach Anspruch 7, wobei die Steuerschaltung so konfiguriert ist, dass sie die Leistungsflusssteuereinheit einschaltet, während die schaltbare Last ausgeschaltet ist, und Strominformationen vom Stromsensor empfängt, die einen ersten Strompegel darstellen.

9. Atmungs- oder Operationsbefeuchter nach Anspruch 8, wobei die Steuerschaltung so konfiguriert ist, dass sie ein Fehlen eines Rohrheizelements oder einen Fehler erfasst, wenn der erste Strompegel außerhalb eines zulässigen Bereichs liegt.

10. Atmungs- oder Operationsbefeuchter nach Anspruch 8 oder Anspruch 9, wobei die Steuerschaltung so konfiguriert ist, dass sie die Leistungsflusssteuereinheit einschaltet, während die schaltbare Last eingeschaltet ist, und Strominformationen vom Stromsensor empfängt, die einen zweiten Strompegel darstellen, wobei vorzugsweise:
die Steuerschaltung so konfiguriert ist, dass sie einen Fehler erfasst, wenn die Differenz zwischen dem ersten und dem zweiten Strompegel außerhalb eines zulässigen Bereichs liegt; oder
die Steuerschaltung so konfiguriert ist, dass sie einen Fehler erfasst, wenn die Differenz zwischen dem ersten Strompegel und einem erwarteten ersten Strompegel oder die Differenz zwischen dem zweiten Strompegel und einem erwarteten zweiten Strompegel außerhalb eines zulässigen Bereichs liegt.

11. Atmungs- oder Operationsbefeuchter nach Anspruch 10, wobei der erwartete erste Strompegel, der erwartete zweite Strompegel oder der zulässige Bereich für die Differenz zwischen dem ersten und dem zweiten Strompegel auf der Basis eines Spannungspegels über die Ausgangsklemmen und einer schaltbaren Lastimpedanz und einer Rohrheizelementimpedanz bestimmt werden.

12. Atmungs- oder Operationsbefeuchter nach Anspruch 10 oder 11, wobei der zulässige Bereich für die Differenz zwischen dem ersten und dem zweiten Strompegel oder die Differenz zwischen dem ersten Strompegel und dem erwarteten ersten Strompegel oder die Differenz zwischen dem zweiten Strompegel und dem erwarteten zweiten Strompegel ein zulässiger Strompegel plus oder minus einer Fehlertoleranz ist.

13. Atmungs- oder Operationsbefeuchter nach einem der Ansprüche 10 bis 12, wobei die Steuerschaltung so konfiguriert ist, dass sie einen Fehler erfasst, wenn weder der erste noch der zweite Strompegel einen Mindestschwellenwert überschreitet.

14. Atmungs- oder Operationsbefeuchter nach einem der Ansprüche 7 bis 13, wobei die Steuerschaltung so konfiguriert ist, dass sie Strominformationen vom Stromsensor empfängt, wenn die Leistungsflusssteuereinheit ausgeschaltet und die schaltbare Last eingeschaltet ist, und einen Fehler erfasst, wenn der Strom einen Niederstromschwellenwert überschreitet.

15. Verfahren zum Überwachen des Betriebs eines Atmungs- oder Operationsbefeuchters, wobei der Atmungs- oder Operationsbefeuchter Ausgangsklemmen (204, 205) zum Liefern von Leistung an ein Rohrheizelement eines Befeuchterversorgungsrohrs aufweist, wenn das Rohrheizelement über die Ausgangsklemmen (204, 205) angeschlossen ist, **dadurch gekennzeichnet, dass**:
das Verfahren vorübergehendes Anschließen einer Dummy-Last über die Ausgangsklemmen, während Leistung an das Rohrheizelement geliefert wird, und Auswerten eines oder mehrerer Leistungsversorgungsattribute umfasst, um einen korrekten Betrieb des Atmungs- oder Operationsbefeuchters zu überwachen.

## Revendications

1. Humidificateur respiratoire ou chirurgical comprenant :
a. des bornes de sortie (204, 205) configurées de sorte qu'un élément chauffant de tube peut être connecté aux bornes de sortie ;
b. des lignes d'alimentation (202, 203) configurées pour fournir de la puissance par l'intermédiaire d'un chemin d'alimentation aux bornes de sortie (204, 205) ;
c. une charge commutable (210) ;
d. un circuit de commande (208) configuré pour commander la commutation de la charge commutable (210) ; et
e. un capteur (209 ; 209, 216) ;
**caractérisé en ce que** :
la charge commutable est connectée électriquement aux bornes de sortie ;
le capteur surveille au moins un attribut de puissance fournie par l'intermédiaire du chemin d'alimentation et fournit des informations d'attribut au circuit de commande ; et
le circuit de commande est configuré pour commander la charge commutable pour fonctionner dans différents états et déterminer le fonctionnement de l'humidificateur respiratoire ou chirurgical sur la base des informations d'attribut pour les différents états.

2. Humidificateur respiratoire ou chirurgical selon la revendication 1, dans lequel le circuit de commande est configuré pour commander la charge commutable pour tester l'humidificateur respiratoire ou chirurgical pendant que de la puissance est fournie à un élément chauffant de tube connecté électriquement aux bornes de sortie.

3. Humidificateur respiratoire ou chirurgical selon la revendication 1 ou la revendication 2, comprenant un dispositif de commande de flux de puissance commandé par le circuit de commande pour faire varier le niveau de puissance fourni par l'intermédiaire du chemin d'alimentation, de préférence dans lequel le dispositif de commande de flux de puissance est un commutateur pouvant être commandé dans le chemin d'alimentation.

4. Humidificateur respiratoire ou chirurgical selon la revendication 3, dans lequel le circuit de commande commande des combinaisons d'état de la charge commutable et du dispositif de commande de flux de puissance pour surveiller le fonctionnement de l'humidificateur respiratoire ou chirurgical en sur la base d'informations d'attribut pour les différentes combinaisons d'état.

5. Humidificateur respiratoire ou chirurgical selon l'une quelconque des revendications précédentes, dans lequel la charge commutable comprend :
a) un commutateur pouvant être commandé, le commutateur pouvant être commandé comprenant de préférence un commutateur à semi-conducteur ; et
b) une impédance, l'impédance comprenant de préférence une résistance ou une impédance réactive.

6. Humidificateur respiratoire ou chirurgical selon l'une quelconque des revendications précédentes comprenant un capteur de tension sur les lignes d'alimentation de puissance, de préférence dans lequel les informations d'attribut comprennent la tension sur les lignes d'alimentation de puissance.

7. Humidificateur respiratoire ou chirurgical selon la revendication 3 ou 4, ou la revendication 5 ou 6 lorsqu'elle dépend de la revendication 3 ou 4, dans lequel le capteur comprend un capteur de courant, de préférence dans lequel le capteur de courant comprend un capteur inductif, à effet Hall ou à résistance shunt, plus préférentiellement dans lequel l'information d'attribut comprend un courant circulant dans le chemin d'alimentation.

8. Humidificateur respiratoire ou chirurgical selon la revendication 7, dans lequel le circuit de commande est configuré pour mettre en marche le dispositif de commande de flux de puissance tandis que la charge commutable est éteinte et recevoir des informations de courant à partir du capteur de courant représentant un premier niveau de courant.

9. Humidificateur respiratoire ou chirurgical selon la revendication 8, dans lequel le circuit de commande est configuré pour détecter une absence d'un élément chauffant de tube ou une erreur si le premier niveau de courant est hors d'une plage permise.

10. Humidificateur respiratoire ou chirurgical selon la revendication 8 ou la revendication 9, dans lequel le circuit de commande est configuré pour mettre en marche le dispositif de commande de flux de puissance tandis que la charge commutable est mise en marche et recevoir des informations de courant à partir du capteur de courant représentant un second niveau de courant, de préférence dans lequel :
le circuit de commande est configuré pour détecter une erreur si la différence entre les premier et second niveaux de courant est hors d'une plage permise ; ou
le circuit de commande est configuré pour détecter une erreur si la différence entre le premier niveau de courant et un premier niveau de courant attendu ou la différence entre le second niveau de courant et un second niveau de courant attendu est hors d'une plage permise.

11. Humidificateur respiratoire ou chirurgical selon la revendication 10, dans lequel le premier niveau de courant attendu, le second niveau de courant attendu ou la plage permise pour la différence entre les premier et second niveaux de courant sont déterminés sur la base d'un niveau de tension aux bornes de sortie et d'une impédance de charge commutable et d'une impédance d'élément chauffant de tube.

12. Humidificateur respiratoire ou chirurgical selon la revendication 10 ou 11, dans lequel la plage permise pour la différence entre les premier et second niveaux de courant ou la différence entre le premier niveau de courant et le premier niveau de courant attendu ou la différence entre le second niveau de courant et le second niveau de courant attendu est un niveau de courant permis plus ou moins une marge d'erreur.

13. Humidificateur respiratoire ou chirurgical selon l'une quelconque des revendications 10 à 12, dans lequel le circuit de commande est configuré pour détecter un défaut si ni le premier ni le second niveau de courant est supérieur à un niveau de seuil minimal.

14. Humidificateur respiratoire ou chirurgical selon l'une quelconque des revendications 7 à 13, dans lequel le circuit de commande est configuré pour recevoir des informations de courant à partir du capteur de courant lorsque le dispositif de commande de flux de puissance est éteint et la charge commutable est allumée, et détecter un défaut si le courant est supérieur à un niveau de seuil de courant bas.

15. Procédé de surveillance du fonctionnement d'un humidificateur respiratoire ou chirurgical, l'humidificateur respiratoire ou chirurgical présentant des bornes de sortie (204, 205) pour alimenter un élément chauffant de tube d'un tube d'alimentation d'humidificateur lorsque l'élément chauffant de tube est connecté aux bornes de sortie (204, 205), **caractérisé en ce que** :
le procédé comprend la connexion temporaire d'une charge fictive aux bornes de sortie tandis que de la puissance est fournie à l'élément chauffant du tube et l'évaluation d'un ou de plusieurs attributs d'alimentation de puissance pour surveiller le bon fonctionnement de l'humidificateur respiratoire ou chirurgical.
